# EUROPEAN PATENT APPLICATION

(11) **EP 2 174 627 A1**
(43) Date of publication of application: **14.04.2010**
(21) Application number: 08778093.8
(22) Date of filing: 11.07.2008
(51) Int. Cl.: A61F 13/15, A61F 13/472, A61F 13/53, A61F 13/534

(54) **ABSORPTIVE ARTICLE AND METHOD OF PRODUCING ABSORPTIVE ARTICLE**

(30) Priority: 30.07.2007 JP 2007197745
(71) Applicant: Uni-Charm Corporation, Ehime 7990111 (JP)
(72) Inventor: NODA, Yuki, Kanonji-shi Kagawa 769-1602 (JP); TSUKUDA, Atsushi, Kanonji-shi Kagawa 769-1602 (JP); KURODA, Kenichiro, Kanonji-shi Kagawa 769-1602 (JP)
(74) Representative: Sperling, Rüdiger
(86) International application number: PCT/JP2008/062578
(87) International publication number: WO 2009/016941

(57) **Abstract**

Occurrence of a protuberance due to swelling of superabsorbent resin is prevented in an absorbent article.

An absorbent article includes: a fluid-permeable surface sheet; a fluid-impermeable back face sheet; and a liquid-absorbent core that is between the surface sheet and the back face sheet and that includes hydrophilic fiber and superabsorbent resin, the liquid-absorbent core having a plurality of through holes formed thereon and high-density regions, the high-density regions being each provided at a position adjacent to each of the through holes, a density of the superabsorbent resin in each of the high-density regions being higher than in another region.

## Description

### Technical Field

The invention relates to an absorbent article, and a method for producing an absorbent article. In particular, the invention relates to an absorbent article including superabsorbent resin, and a method for producing the absorbent article.

### Background Art

There has been already known absorbent articles having an absorbent body (absorbent layered body) including superabsorbent resin. These absorbent articles are used for absorbing a certain fluid such as menstrual blood, and the absorbed fluid is held by superabsorbent resin in their own absorbent body. There are some absorbent articles having a portion in which the density of superabsorbent resin in their own absorbent body is higher than in other portions in order to appropriately absorb fluid (for example, see JP-T-9-504207).

### Disclosure of Invention

### Problem to be Solved by the Invention

It is in the nature of superabsorbent resin to swell when fluid is held therein. Therefore, if a portion in which the density of superabsorbent resin is higher than in other portions exists in an absorbent body, there is a risk that a portion in which the swelling superabsorbent resin exists becomes a protuberance and protrudes from a surface of an absorbent article. As a result thereof, the protuberance occurring in the absorbent article gives foreign-body sensation to a wearer of the absorbent article.

The invention has been contrived in view of the above and other problems, and an advantage thereof is to provide an absorbent article that prevents, in the absorbent article, occurrence of a protuberance caused by swelling of superabsorbent resin, and a method for producing the absorbent article.

### Means for Solving the Problem

In order to solve the above-described problems, a principal aspect of the invention is an absorbent article, including: a fluid-permeable surface sheet; a fluid-impermeable back face sheet; and a liquid-absorbent core that is between the surface sheet and the back face sheet and that includes hydrophilic fiber and superabsorbent resin, the liquid-absorbent core having a plurality of through holes formed thereon and high-density regions, the high-density regions being each provided at a position adjacent to each of the through holes, a density of the superabsorbent resin in each of the high-density regions being higher than in another region.

Features of the invention other than the above will become clear by reading the description of the present specification with reference to the accompanying drawings.

### Effects of the Invention

With the invention, it is possible to provide an absorbent article that prevents, in the absorbent article, occurrence of a protuberance caused by swelling of superabsorbent resin, and a method for producing the absorbent article.

### Brief Description of Drawings

[FIG. 1] This is a schematic plan view showing the configuration of an absorbent article.
[FIG. 2] This is a schematic plan view of an absorbent body, showing a skin-contacting side of the absorbent body.
[FIG. 3] This is a diagram showing a cross-section taken along A-A in FIG. 2.
[FIG. 4] This is a chart showing a production flow of the absorbent article.
[FIG. 5] This is a flowchart of an absorbent-body-production step.
[FIG. 6] This is a diagram schematically showing how the absorbent body is being produced in the absorbent-body-production step.
[FIG. 7] This is a diagram showing a mesh pattern for forming an absorbent body material by accumulating pulverized pulp.
[FIG. 8] This is a cross-sectional view for illustrating a high-density region having a two-layer structure.
[FIG. 9] This is a cross-sectional view for illustrating a high-density region having a one-layer structure.
[FIG. 10] These are diagrams for illustrating a method of producing the absorbent body material using an air-laid sheet. FIG. 10A is a cross-sectional view of an unprocessed air-laid sheet. FIG. 10B is a cross-sectional view showing a base material having a hole section and a concave section formed therein. FIG. 10C is a cross-sectional view showing an absorbent body material to whose concave section superabsorbent polymer has been supplied. FIG. 10D is a cross-sectional view showing an absorbent body in which thin paper and the absorbent body material are integrated.

### List of Reference Numerals

- 1: absorbent article,
- 10: absorbent body (liquid-absorbent core),
- 10a: swelling section, 11 thin paper,
- 12: absorbent body material, 12a thick wall section,
- 13: high-density region,
- 13a: superabsorbent polymer accumulated layer,
- 13b: pulp accumulated layer, 15 base material,
- 15a: hole section (through hole),
- 15b: concave section, 20 surface sheet,
- 20a: deep channel section,
- 25: side sheet, 30 back face sheet, 32 holding section,
- 40: suction drum (drum),
- 42: pulp opening-and-supplying apparatus,
- 44: pulp scraping mechanism, 46 pulp supplying section,
- 48: mesh pattern (mold), 48a projection section,
- 48b: deep bottom section, 48c bottom section,
- 50: thin-paper supplying section,
- 52: superabsorbent-polymer supplier,
- 54: air-laid sheet, 60 suction conveyor,
- P: pulverized pulp (hydrophilic fiber),
- S: superabsorbent polymer (superabsorbent resin),

### Best Mode for Carrying Out the Invention

At least the following matters will be disclosed in the description in the present specification and the accompanying drawings.

An absorbent article, including: a fluid-permeable surface sheet; a fluid-impermeable back face sheet; and a liquid-absorbent core that is between the surface sheet and the back face sheet and that includes hydrophilic fiber and superabsorbent resin, the liquid-absorbent core having a plurality of through holes formed thereon and high-density regions, the high-density regions being each provided at a position adjacent to each of the through holes, a density of the superabsorbent resin in each of the high-density regions being higher than in another region.

With this absorbent article, the high-density regions in which the density of the superabsorbent resin is higher than in the other region are each provided at the position adjacent to each of the through holes. Therefore, even if the superabsorbent resin absorbs fluid and swells so that a volume of the superabsorbent resin increases, it is possible to accommodate the increased volume in the through hole. Accordingly, it is possible to prevent a protuberance from occurring on an outer surface of the absorbent article when the superabsorbent resin absorbs fluid and swells.

In such an absorbent article, it is preferable that the liquid-absorbent core is substantially rectangular, and a plurality of the through holes are provided in both a longitudinal direction and a width direction of the liquid-absorbent core.
When the absorbent article absorbs fluid, superabsorbent resin that swells exists in the through hole. The swelling superabsorbent resin becomes unlikely to absorb fluid, and in a region in which the swelling superabsorbent resins connect, it becomes impossible for fluid to permeate. Accordingly, a plurality of the through holes are provided in both the longitudinal direction and the width direction of the liquid-absorbent core. Thereby, it is possible to realize a configuration in which, even if the superabsorbent resin of the high-density region that is provided adjacent to the through hole swells, the swelling superabsorbent resins do not connect due to hydrophilic fiber existing between the swelling superabsorbent resins. Accordingly, fluid widely permeates the hydrophilic fiber between the swelling superabsorbent resins; and a plurality of the superabsorbent resins absorbs the fluid, the superabsorbent resins are provided apart in the longitudinal direction and are provided apart in the width direction of the liquid-absorbent core. Thereby, it is possible to provide an absorbent article having high absorbency.

In such an absorbent article, it is preferable that the high-density region is adjacent to the through hole on at least either one of both sides of the through hole in the longitudinal direction of the liquid-absorbent core.

With this absorbent article, the high-density region is adjacent to the through hole on at least either one of both sides of the through hole in the longitudinal direction of the liquid-absorbent core. Therefore, it is possible to position a plurality of the formed high-density regions in such a manner as not to be close to each other. Accordingly, since, even if the superabsorbent resin swells, the swelling superabsorbent resins do not connect, it is possible to suppress absorbency deterioration caused by inhibiting the penetration of fluid.

Further, a method for producing an absorbent article, including: a concave-section-forming step in which hydrophilic fiber is supplied to a mold having a projection section that projects from a bottom section and that is used to form a through hole extending in a front-back face direction, from a certain direction with respect to the mold, and in which a hollowed concave section is formed on an opposite side to a side of the projection section on which the hydrophilic fiber is supplied; and a supplying step in which superabsorbent resin is supplied to the concave section.

With this method for producing an absorbent article, the projection section projected from the bottom section prevents the hydrophilic fiber supplied from the certain direction, from being supplied to the opposite side to the side of the projection section on which the hydrophilic fiber is supplied. Therefore, supplying the hydrophilic fiber to the mold from the certain direction is all that is required to allow the concave section to be easily formed on the opposite side to the side of the projection section on which the hydrophilic fiber is supplied. Since the through hole is formed at a position of the projection section when being removed from the mold, it is possible to form the concave section at a position adjacent to the through hole by only supplying the hydrophilic fiber to the mold from the certain direction.

In such a method for producing an absorbent article, it is preferable that the mold is recessed on an outer circumferential face of a drum that rotates, and the hydrophilic fiber is supplied from above the drum to the mold whose orientation changes with rotation of the drum.

With this method for producing an absorbent article, the hydrophilic fiber is supplied from above the drum on whose outer circumferential face the mold is provided. Therefore, it is possible to supply the hydrophilic fiber to the mold with changing the orientation of the mold by rotation of the drum. That is, it is possible to supply the hydrophilic fiber with changing by rotate of the drum a direction in which the hydrophilic fiber is supplied to the mold.

In such a method for producing an absorbent article, it is preferable that after the concave-section-forming step; the mold is moved by rotation of the drum to a position at which the superabsorbent resin can be supplied to the concave section from above.

With this method for producing an absorbent article, rotation of the drum allows the superabsorbent resin to be surely supplied to the concave section that is formed in the concave-section-forming step and that is adjacent to the through hole.

### Absorbent Article according to Present Embodiment

### Overall Configuration of Absorbent Article

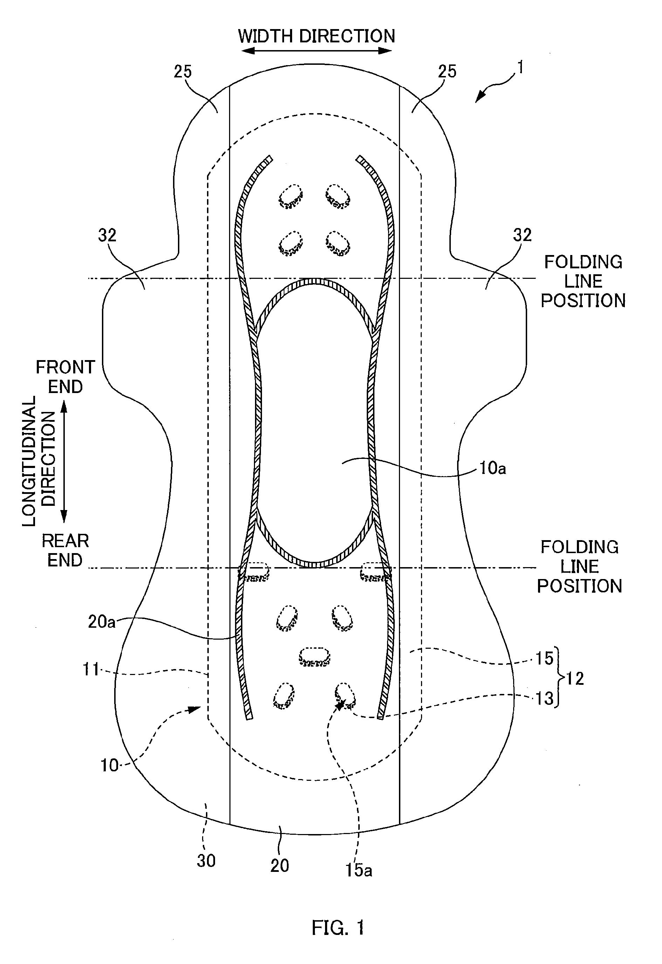

First, as an example of an absorbent article of the present embodiment, using a sanitary napkin as an example (hereinafter referred to as an absorbent article 1), an example configuration of the absorbent article 1 will be described with reference to FIG. 1. FIG. 1 is a schematic plan view of the absorbent article 1. In FIG. 1, a longitudinal direction and a width direction of the absorbent article 1 are indicated by the arrows. In the description below, among surfaces of the absorbent article 1, a face that contacts a body of a wearer of the absorbent article 1 is referred to as a skin-contacting face, and a face that contacts an undergarment is referred to as a non-skin-contacting face. Further, an end that is positioned on a front side of a wearer when worn is referred to as a front end, and an end that is position on a rear side is referred to as a rear end. Note that FIG. 1 shows a skin-contacting side of the absorbent article 1.

The absorbent article 1 has a shape elongated in a predetermined direction as shown in FIG. 1.
Further, the absorbent article 1 includes: an absorbent body 10 as a substantially rectangular liquid-absorbent core for absorbing fluid such as menstrual blood; a fluid-permeable surface sheet 20 covering a surface of the absorbent body 10 on the skin-contacting side; a side sheet 25 disposed of both ends of the absorbent body 10 in a longitudinal direction on the skin-contacting side of the absorbent body 10; and a fluid-impermeable back face sheet 30 provided on a non-skin-contacting side of the absorbent body 10. Besides, the absorbent article 1 can be fold on a folding line that is along the width direction. The absorbent article 1 is folded in three on a certain folding line position (indicated by the double-dotted chained line in FIG. 1) when wrapped as a product. Hereinafter, in the longitudinal direction of the absorbent article 1, a section on a side closer to the front end from the folding line position of the front end side is defined as a front end section, a section on a side closer to the rear end from the folding line position of the rear end side is defined as a rear end section, and a section between the two folding line positions is defined as a central section.

The longitudinal direction of the absorbent body 10 is arranged along the longitudinal direction of the absorbent article 1. Besides, in a central region of the absorbent body 10, a swelling section 10a that swells toward the skin-contacting side is formed. The swelling section 10a is formed having a substantially oval shape. When a wearer wears the absorbent article 1, the swelling section 10a contacts the wearer at a groin (that is, around the menstrual blood discharge opening of the wearer) via the surface sheet 20 while deforming according to a shape of the groin.

Also, the absorbent body 10 according to the present embodiment consists of thin paper 11 such as tissue paper and an absorbent body material 12. The absorbent body material 12 has a function to absorb and hold fluid that has penetrated from the surface sheet 20 side to the absorbent body 10 side. The absorbent body material 12 has approximately the same external shape as the absorbent body 10. The thin paper 11 is an example of "covering member", and a sheet covering the absorbent body material 12 in such a manner as to wrap the material. In order to integrate the thin paper 11 and the absorbent body material 12 covered by the thin paper 11, a certain portion is embossed (compressed), so that embossing (hereinafter referred to as absorbent body embossing) is formed in the embossed portion. The absorbent body 10 will be described in detail later.

The surface sheet 20 is a fluid-permeable sheet member. The surface sheet 20 is formed of woven or nonwoven fabric, a perforated plastic sheet or the like that has been formed of natural fiber such as pulp or cotton, cellulose fiber such as rayon, or thermoplastic hydrophobic fiber such as polyethylene or polypropylene. The surface sheet 20 is included in the central region of the absorbent article 1 in the width direction. The surface sheet 20 has a width slightly wider than the absorbent body 10 in the width direction and approximately the same length as the back face sheet 30 in the longitudinal direction, and covers an entire surface of the absorbent body 10.

The back face sheet 30 is a thermoplastic, fluid-impermeable sheet made of polyethylene, polypropylene, or the like. The back face sheet 30 is formed sufficiently wider than the absorbent body 10, and an entire circumference of an outer edge section thereof is positioned to the outside of an outer edge section of the absorbent body 10. Also, on both sides in the width direction, holding sections 32 are formed on a front end side of the central section, extending to the outside in the width direction. Adhesive is applied to the holding sections 32 on the non-skin-contacting side, and, when wearing the absorbent article 1, are fixed with the adhesive to the outside of the undergarment, in a state folded back to the non-skin-contacting side. Further, the back face sheet 30 according to the present embodiment is a thermoplastic, fluid-impermeable sheet made of polyethylene, polypropylene, or the like; but it is also acceptable to use a sheet member in which thin paper, nonwoven fabric, or the like has been layered and that includes thermoplastic, fluid-impermeable sheet.

The side sheets 25 are appropriate nonwoven fabrics, such as air-through nonwoven fabric or spun-bonded nonwoven fabric formed with synthetic resin fiber, or nonwoven fabric made up of spun-bonded/melted-blown/spun-bonded layers. The side sheets 25 are provided on both end sections of the absorbent article 1 in the width direction, in a state in which the side sheets 25 overlap both end sections of the surface sheet 20 in the width direction.

In the absorbent article 1 configured as mentioned above, the skin-contacting face of the absorbent body 10 and the surface sheet 20 are joined with hot-melt adhesive. The skin-contacting face of the absorbent body 10 and the surface sheet 20 are joined more firmly with a deep channel section 20a formed by a deep channel embossing process of pressing in the thickness direction using a high-temperature pressing member.

As shown in FIG. 1, the deep channel section 20a according to the present embodiment consists of a section surrounding the swelling section 10a, and a section extending toward the front end and the rear end from both sides of that section surrounding the swelling section 10a in the width direction. Besides, in the deep channel section 20a, a shallow bottom section and a deep bottom section whose depth are different from each other are alternately arranged along the deep channel section 20a. Due to the deep channel section 20a configured in this manner, when a central section of the absorbent article 1 in the longitudinal direction is bent in such a manner as to make its planar width smaller, both end sections of the absorbent article 1 in the longitudinal direction is easy to be bent together with the central section. That is, the deep channel section 20a serves as a bend-inducing section that facilitates three-dimensional bending in order to bring a portion that corresponds to the above-mentioned swelling section 10a into closer contact with the body when the absorbent article 1 is worn. Further, the deep channel section 20a has a function to, when menstrual blood or the like has flowed into the deep channel section 20a, suppress scattering of the menstrual blood or the like by facilitating penetration to a portion that has been compressed with high density (that is, a deep bottom section). Note that, in the present embodiment, the shallow bottom section and the deep bottom section are alternately arranged in the deep channel section 20a, but this invention is not limited thereto. For example, the channel depth in the deep channel section 20a may be uniform.

Further, the back face sheet 30 is joined with hot-melt adhesive to the non-skin-contacting side of the superposed absorbent body 10 and surface sheet 20. Besides, the side sheet 25. is joined with hot-melt adhesive to the skin-contacting side of the absorbent body 10, from a position slightly overlapping both sides of the absorbent body 10 in the width direction to ends of the back face sheet 30. At positions where the back face sheet 30, the absorbent body 10, the surface sheet 20, and the side sheet 25 are superposed, an embossing process is performed with a pressing member heated to a low temperature, and thus more firmly joining the absorbent body 10, the surface sheet 20, the side sheet 25, and the back face sheet 30. Furthermore, a round sealing process is performed in which an outer edge of the absorbent article 1 is hot-melt bonded at a low temperature. As mentioned above, the absorbent body 10, the surface sheet 20, the side sheet 25, and the back face sheet 30 are joined by an embossing process, or with hot-melt adhesive, or the like. As a result thereof, the absorbent body 10 is accommodated within a space formed by the surface sheet 20, the side sheet 25, and the back face sheet 30.

### Structure of Absorbent Body

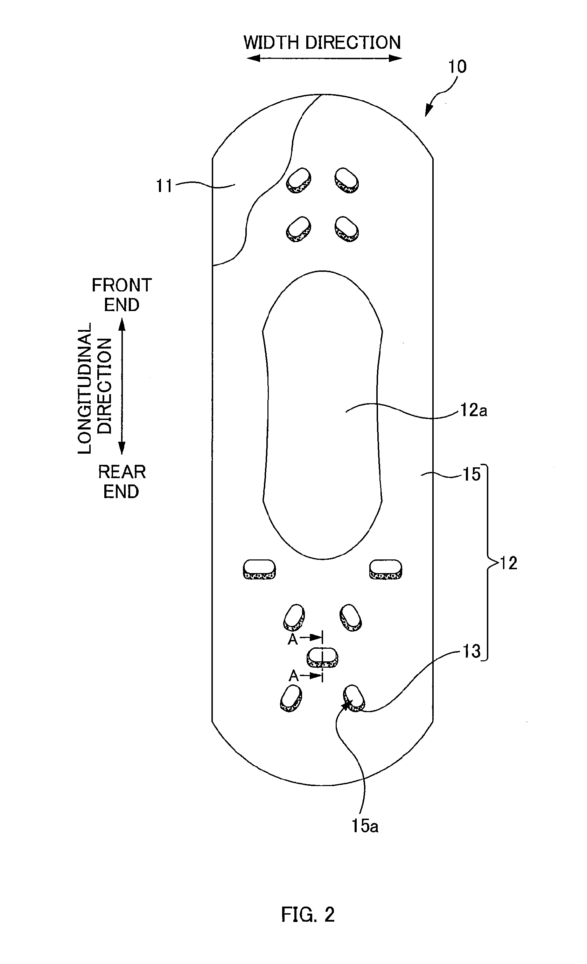

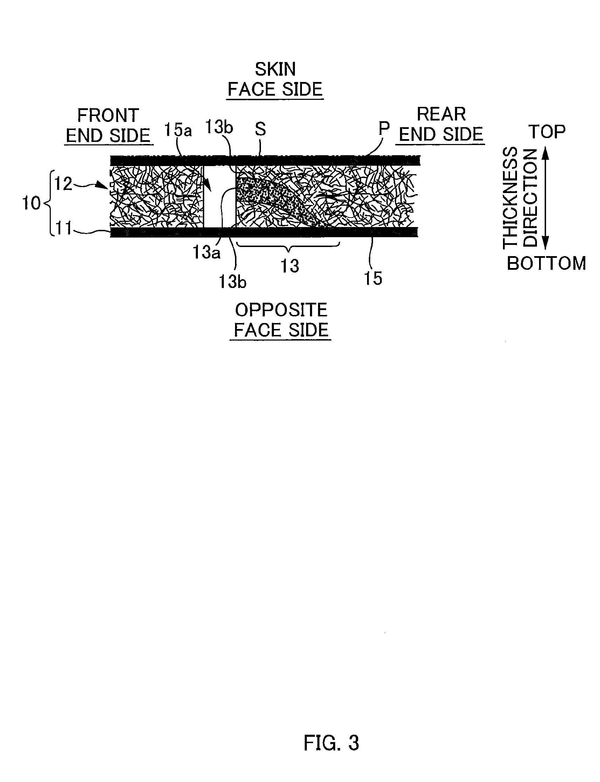

Next, the structure of the absorbent body 10 of the present embodiment is described with reference to FIGS. 1 to FIG. 3. FIG. 2 is a plan view of a schematic diagram showing the absorbent body, and shows the skin-contacting side of the absorbent body 10. FIG. 3 is a diagram showing a cross-sectional structure of a through hole and a high-density region, and shows a cross section taken along A-A in FIG. 2. Besides, in FIG. 2, the longitudinal direction and the width direction of the absorbent body 10 are indicated by the arrows. In FIG. 3, the vertical direction, that is, a thickness direction of the absorbent body 10 is indicated by the arrow. Here, a direction along a plane defined by the longitudinal direction and the width direction of the absorbent body 10 is a direction intersecting the thickness direction.

The absorbent body 10 is an approximately sheet-shaped member elongated in a certain direction as shown in FIG. 2, and consists of the thin paper 11 and the absorbent body material 12, as mentioned above.

The absorbent body material 12 has approximately the same external shape as the absorbent body 10. The absorbent body material 12 is attached to the inside of the absorbent article 1 in such a manner that a longitudinal direction and a width direction of the absorbent body material 12 are respectively along the longitudinal direction and the width direction of the absorbent article 1. A portion that is positioned in the center in the longitudinal direction and in the center of the width direction of the absorbent body material 12 serves as a the swelling section 10a of the absorbent body 10. A thick wall section 12a that is thicker than other portions is formed on that portion.

This absorbent body material 12 consists of pulverized pulp P (pulp that has been pulverized into fibrous state) as an example of "hydrophilic fiber", and granular superabsorbent polymer S as an example of "superabsorbent resin". The pulverized pulp P is accumulated in sheet-like form, and the superabsorbent polymer S is arranged in such a manner as to be densely gathered in some portions of the absorbent body material 12.

The structure of the absorbent body material 12 of the present embodiment is specifically described with reference to FIGS. 2 and 3. As shown in FIG. 2, the absorbent body material 12 includes the pulverized pulp P and the superabsorbent polymer S that are for absorbing fluid. In the absorbent body material 12, hole sections 15a as a plurality of through holes are provided. In the absorbent body 10 wrapped with the thin paper 11, an absorbent material region in which absorbent material including the pulverized pulp P and the superabsorbent polymer S exists serves as the absorbent body material 12. The hole section 15a is formed in an oval shape on a plane defined by the longitudinal direction and the width direction of the absorbent body material 12. In a front end side and a rear end side of the absorbent body 10, the plurality of hole sections 15a are positioned apart from each other in directions intersecting the thickness direction, that is, the plurality of hole sections 15a are provided in the longitudinal direction of the absorbent body 10 and the plurality of hole sections 15a are provided in the width direction.

In the absorbent body material 12, at a position adjacent to each hole section 15a in a direction intersecting the thickness direction, a high-density region 13 is provided in which the density of superabsorbent polymer S is higher than in another region. Here, sections of the absorbent body material 12 other than the high-density region 13 are referred to as a base material 15 in the description below. Further, each high-density region 13 is adjacent to the hole section 15a in a specific direction (here, a rear end direction), and are positioned apart from each other in the same manner as the hole section 15a. In the vicinity of a portion where the hole section 15a and the high-density region 13 adjacent thereto exists, pulverized pulp P is accumulated in an approximately uniform state. Note that, in a surface of the absorbent body material 12 (a surface of the skin-contacting side or the non-skin-contacting side), a ratio of an area of portions where the hole section 15a and the high-density region 13 adjacent thereto exists is set to 5% or more to a total area of the absorbent body material 12 (more preferably, 5 to 70% or more; it is particularly preferable to set the ratio to 10 to 40%).

As shown in FIG. 3, in the portion where the hole section 15a and the high-density region 13 adjacent thereto exists according to the present embodiment, the absorbent material region in which absorbent materials such as pulverized pulp P and superabsorbent polymer S are accumulated (the absorbent body material 12) and the hole section 15a in which absorbent material does not exist are provided adjacent to each other in the longitudinal direction. In the absorbent body material 12, the high-density region 13 that has a three-layer structure in the thickness direction of the absorbent body material 12 is provided on a rear end side of the hole section 15a, in the longitudinal direction of the absorbent body material 12. The high-density region 13 includes a pulp accumulated layer 13b in which the pulverized pulp P is accumulated, and a superabsorbent polymer accumulated layer 13a in which superabsorbent polymer S (indicated by letter S in FIG. 3, etc.) is accumulated. The superabsorbent polymer accumulated layer 13a is provided between two pulp accumulated layers 13b that are provided on the skin-contacting side and the non-skin-contacting side.

Regarding all of a plurality of the portions where the hole section 15a and the high-density region 13 adjacent thereto exists and that are positioned apart from each other in the absorbent body material 12, the high-density region 13 is arranged on the rear end side of the hole section 15a in the longitudinal direction. In addition, a part of the superabsorbent polymer S accumulated in the superabsorbent polymer accumulated layer 13a is exposed to the hole section 15a. Here, while the pulverized pulp P and the superabsorbent polymer S exist in the absorbent body material 12 of the present embodiment, the superabsorbent polymer S in the base material 15 is contained only to the extent that the polymer is mixed in production processes. Therefore, a rate of superabsorbent polymer S contained in the base material 15 is extremely smaller than in the high-density region 13. On the other hand, since the superabsorbent polymer S is intentionally supplied to the high-density region 13, the high-density region 13 is a region in which the density of the superabsorbent polymer S is high comparing to the base material 15. The configuration of the high-density region 13 is attributed to a producing method. Therefore, it will be described in a section of the producing method.

As mentioned above, since the absorbent body material 12 has the hole sections 15a, the vicinity of the hole section 15a has lower rigidity than a portion apart from the hole section 15a. In the present embodiment, as shown in FIG. 1, the hole section 15a exists at a position corresponding to a folding line position when folding the absorbent article 1. In other words, the hole section 15a exists at a position where is on a crease that is made when the absorbent article 1 is folded. Since the hole section 15a having lower rigidity is on the crease in this manner, it is possible to easily fold the absorbent article 1. In addition, since a longitudinal direction of the hole section 15a existing on the crease is along the crease, it is possible to more easily fold the absorbent article 1.

The thin paper 11 has fluid permeability, and is a sheet with perforations that are smaller than particles of superabsorbent polymer S. The thin paper 11 has a function to prevent superabsorbent polymer S from leaking outside of the thin paper 11. Furthermore, the thin paper 11 has a function to prevent the accumulated pulverized pulp P from dropping outside of the thin paper 11.

In the absorbent bodies 10 of the present embodiment, a plurality of the high-density regions 13 in which the density of the superabsorbent polymer S is high are formed adjacent to the hole section 15a that are positioned apart from each other. Among a plurality of portions in which the high-density region 13 is adjacent to the hole section 15a, the pulverized pulp P is mainly accumulated. That is, fluid passing through the surface sheet 20 and the thin paper 11 penetrates into the accumulated pulverized pulp P so as to scatter and be absorbed with the pulverized pulp P, or is absorbed with the superabsorbent polymer S. At this stage, in the high-density region 13, the superabsorbent polymer S holds the fluid and swells, and the increased volume due to the swelling extends to the adjacent hole section 15a in which there is no obstacle. In addition, since the high-density region 13 has three-layer structure in which the superabsorbent polymer accumulated layer 13a is located substantially at the center in the thickness direction of the absorbent body material 12, the configuration makes it possible that the superabsorbent polymer S extending to the hole section 15a is unlikely to protrude toward any of the skin-contacting face and non-skin-contacting face.

### Method for Producing Absorbent Article

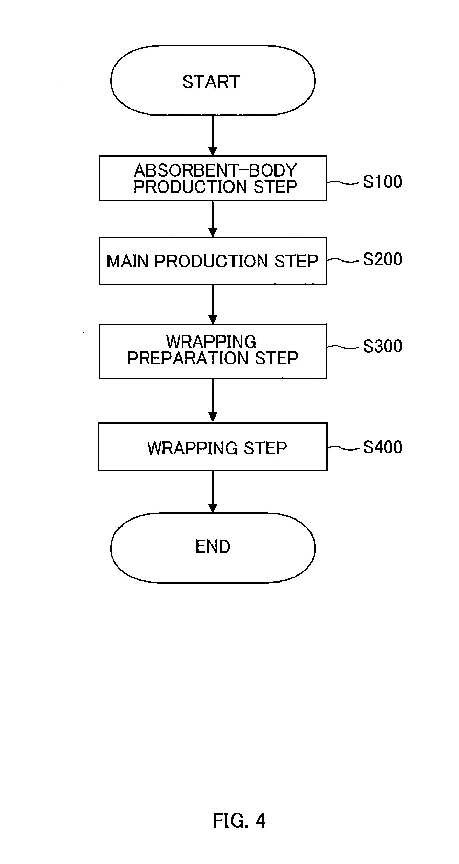

Next, a method for producing the absorbent article 1 according to the present embodiment is described with reference to FIG. 4. FIG. 4 is a chart showing a production flow of the absorbent article 1. The method for producing the absorbent article 1 includes: an absorbent-body-production step S100 in which the absorbent body 10 is produced; a main production step S200 in which the absorbent article 1 is produced by joining the absorbent body 10 produced in the absorbent-body-production step S100, the surface sheet 20, the side sheet 25, and the back face sheet 30; a wrapping preparation step S300 in which the absorbent article 1 is prepared for wrapping; and a wrapping step S400 in which the absorbent article 1 is wrapped. In the present embodiment, the above steps are executed while materials and products of the absorbent article 1 are transported by a transporting apparatus such as a conveyor. The absorbent-body-production step S100 in the production flow of the absorbent article 1 is described below.

### Absorbent-Body-Production Step

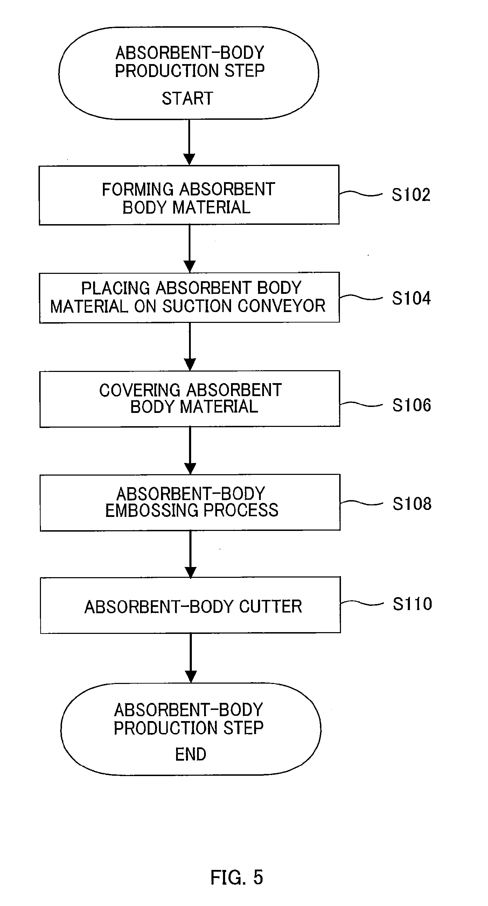

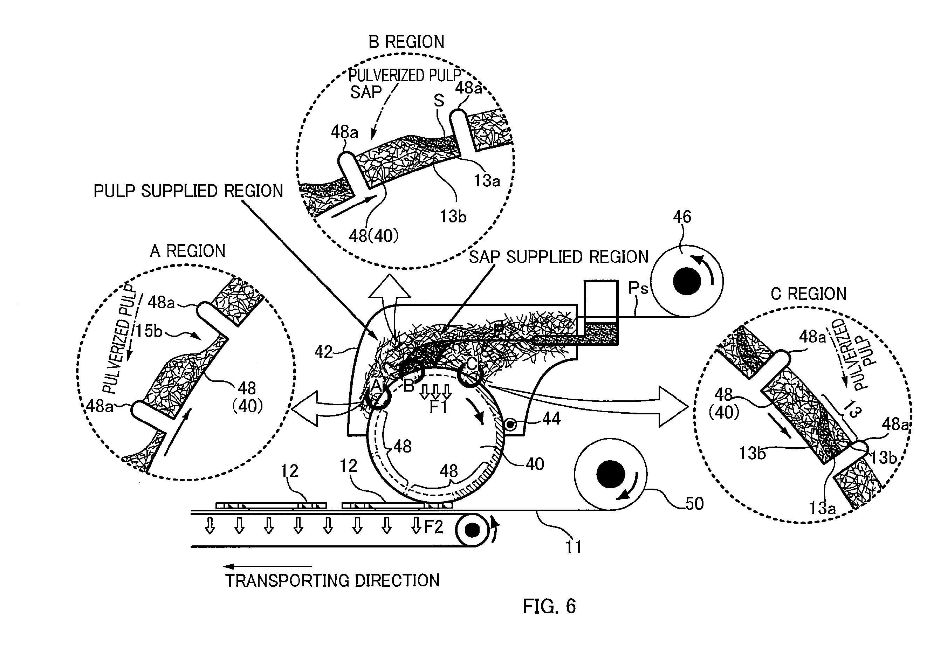

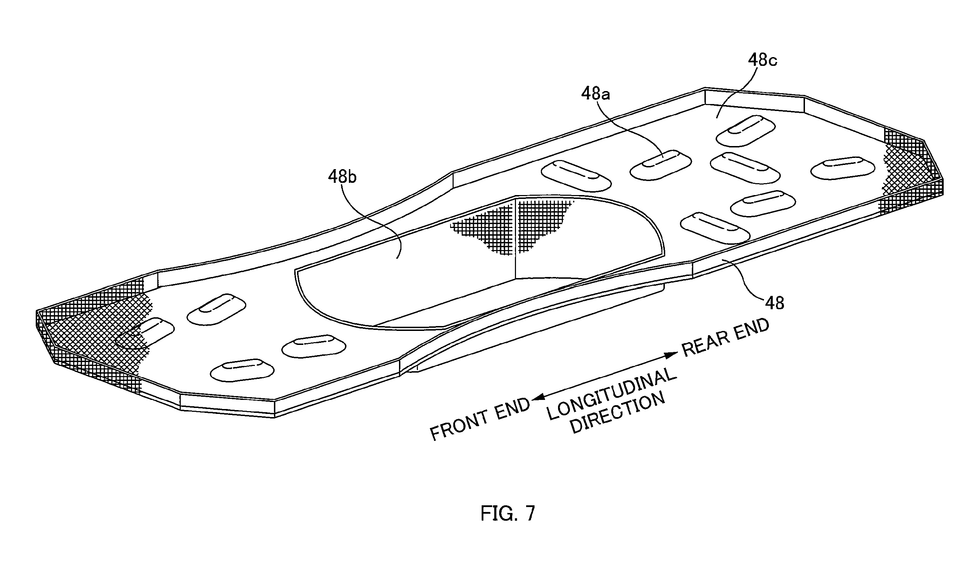

The absorbent-body-production step S100 is described with reference to FIGS. 5 to 7. FIG. 5 is a flowchart of the absorbent-body-production step S100. FIG. 6 is a diagram schematically showing how the absorbent body 10 is being produced in the absorbent-body-production step S100. FIG. 7 is a diagram showing a mesh pattern 48 that is used as an example of a "mold" for forming the absorbent body material 12 by accumulating the pulverized pulp. Note that, for the sake of description, FIG. 6 schematically shows an absorbent body material broken out in such a manner as hole sections and concave sections appear.

As shown in FIG. 5, the absorbent-body-production step S100 starts from step S102 for forming the absorbent body material 12. In the present embodiment, the absorbent body material 12 is formed by accumulating the pulverized pulp P and the superabsorbent polymer S in the mesh pattern 48 shown in FIG. 7.

The mesh pattern 48 has a mesh-like bottom section 48c, and is a metal mold having a shape corresponding to a shape of the above-mentioned absorbent body material 12. That is, the mesh pattern 48 has an external shape elongated in a certain direction. As shown in FIG. 7, projection sections 48a whose horizontal shape is oval and whose vertical cross-sectional shape is substantially trapezoidal are positioned apart from each other on the bottom section 48c of the mesh pattern 48. Besides, in a portion that is in a central section of the absorbent body material 12 in the longitudinal direction and that corresponds to a central region in the width direction, a deep bottom section 48b for forming the thick wall section 12a of the absorbent body material 12 is provided. A plurality of the mesh patterns 48 are provided on an outer circumferential face of a suction drum 40 that rotates. FIG. 7, for the sake of illustration in the figure, shows that only a part of the bottom surface is mesh and the bottom surface is flat.

Further, outside the mesh pattern 48, a suction apparatus (not shown) is provided that sucks air into the suction drum 40. When air is sucked by the suction apparatus in such a manner as to pass through the bottom section 48c of the mesh pattern 48, pulverized pulp P and superabsorbent polymer S that fall from above the suction drum 40 due to suction force are sucked into the mesh pattern 48. Then, the pulverized pulp P is accumulated in the mesh pattern 48, and the layering stops until the pulp reaches a certain thickness.

During movement of the mesh pattern 48 having the above-mentioned configuration while changing its orientation in conjunction with rotation of the suction drum 40, pulverized pulp P and superabsorbent polymer S are accumulated. As a result thereof, the absorbent body material 12 is formed. That is, since the absorbent body material 12 is formed by molding, it becomes possible to form the absorbent body material 12 having a desired shape by changing a shape of the mesh pattern 48. In other words, it is possible to mold the absorbent body material 12 in a desired shape. Note that, in the mesh pattern 48 according to the present embodiment, the deep bottom section 48b for forming the thick wall section 12a is provided. However, the invention is not limited thereto. For example, a section located at the center in the longitudinal direction and the center in the width direction of the mesh pattern 48 may be a flat surface, and a flat absorbent body material without the thick wall section 12a may be formed.

Further, in the absorbent body material 12, the oval hole sections 15a are positioned apart from each other as through holes. The hole section 15a is formed by a projection section 48a that projects from the bottom section 48c of the mesh pattern 48. That is, when the pulverized pulp P is caused to be accumulated in the mesh pattern 48, the pulverized pulp P is accumulated in such a manner as to avoid a section corresponding to the projection section 48a (in other words, in such a manner as to cause the pulverized pulp P not to enter into the projection section 48a), so that the hole section 15a is formed at a portion corresponding to the projection section 48a. In the present embodiment, each side face of the projection section 48a is mesh, but each side face of the projection section 48a may be covered with tape, resin, or the like in order to prevent the pulverized pulp from entering into the hole section 15a. In addition, if the mesh pattern 48 has a flat bottom, the projection section 48a individually formed of rubber, resin, or the like may be attached to the bottom section 48c of the mesh pattern 48.

Step S102 in which the absorbent body material 12 is formed with the mesh pattern 48 is described with reference to FIG. 6 in more detail. The mesh pattern 48 includes the suction apparatus therein. The mesh pattern 48 is arranged on the outer circumference of the suction drum 40 that is rotatable in a certain rotating direction (a direction indicated with the arrow in FIG. 6), in such a manner as the longitudinal direction of the mesh pattern 48 is along a rotational direction of the suction drum 40. And, a portion, of the mesh pattern 48, that forms the front end side of the absorbent body material 12 is positioned frontward in the rotational direction.

Each mesh pattern 48 is arranged on the circumferential face of the suction drum 40 with an opening of the mesh pattern 48 facing outside. While air around the suction drum 40 is sucked by the suction apparatus to the inside of the suction drum 40 via the mesh pattern 48 (that is, in a direction indicated by letter F1 in FIG. 6), the mesh pattern 48 rotates together with the suction drum 40 in an integrated manner. On the other hand, above the suction drum 40, a pulp opening-and-supplying apparatus 42 is provided that is for pulverizing and opening sheet-like pulp (indicated by letter Ps in FIG. 6) and supplying the result. When a pulp supplying section 46 supplies the sheet-like pulp Ps to the pulp opening-and-supplying apparatus 42, the sheet-like pulp Ps is pulverized and opened inside the pulp opening-and-supplying apparatus 42, to produce pulverized pulp P. As shown in FIG. 6, the produced pulverized pulp P is supplied in such a manner as to fall from above the suction drum 40 toward the suction drum 40, and is collected into the mesh pattern 48 arranged on the outer circumference of the suction drum 40 due to suction force of the suction apparatus. At this time, in the present embodiment, a setting is made such as the pulverized pulp P supplied by the pulp opening-and-supplying apparatus 42 is supplied at a region in which a side where the mesh pattern 48 moves upward when the suction drum 40 rotates is wider than a side where the mesh pattern 48 moves downward, with respect to the center of the suction drum 40. The pulverized pulp P is discharged from upper right to left of the suction drum 40 in FIG. 6, and is supplied in such a manner as to be pulled toward the suction drum 40 due to weight of the pulverized pulp P itself and the sucked air.

In pulverized-pulp supplied regions where pulverized pulp P is supplied from above the suction drum 40, at a position where an orientation of the mesh pattern 48 is in such a manner as its opened side faces substantially upward with rotation of the suction drum 40, a superabsorbent-polymer supplied region is provided that superabsorbent polymer S can be supplied by a superabsorbent-polymer supplier 52. The superabsorbent-polymer supplier 52 is an apparatus that scatters the superabsorbent polymer S from above to a certain region of the suction drum 40. In the superabsorbent-polymer supplied region, supplying and non-supplying of the superabsorbent polymer S can be controlled based on positional information or rotational timing of the suction drum 40.

When the mesh pattern 48 that moves upward with rotation of the suction drum 40 reaches the supplied region of the pulverized pulp P, the pulverized pulp P starts to accumulate in the mesh pattern 48 (A region in FIG. 6). At this stage, the pulverized pulp P always accumulates with the suction drum 40 rotating. However, since the orientation of the mesh pattern 48 moving upward with rotation of the suction drum 40 slants, the pulverized pulp P falling from upper right to the slanting mesh pattern 48 does not accumulate at a portion below the projection section 48a of the mesh pattern 48. That is, pulverized pulp P supplied from a direction substantially opposite a movement direction of the mesh pattern 48 is interfered with. As a result thereof, while remaining a region in which the pulverized pulp P does not accumulate on a side opposite, with respect to the projection section 48a, a side where the pulverized pulp P is supplied, the suction drum 40 is rotating and the orientation of the mesh pattern 48 is changing. When the slant of the mesh pattern 48 becomes smaller with rotation of the suction drum 40, pulverized pulp P starts to accumulate in the region opposite, with respect to the projection section 48a, the side where the pulverized pulp P is supplied. At this stage, on a portion positioned below the projection section 48a of the mesh pattern 48, that is, on a portion adjacent to the projection section 48a on a rear end side of the absorbent body material 12, a concave section 15b is formed in which an amount of accumulated pulverized pulp P is less than in other portions. The suction drum 40 further rotates, and the opened side of the mesh pattern 48 faces substantially upward. When the concave section 15b reaches the region where the superabsorbent polymer S is supplied, the superabsorbent polymer S is supplied to the concave section 15b (B region in FIG. 6). At this stage, the superabsorbent polymer accumulated layer 13a is layered in the concave section 15b. Besides, the superabsorbent polymer S is slightly supplied to regions other than the concave section 15b because the superabsorbent-polymer supplier 52 is an apparatus that scatters superabsorbent polymer S to a certain region but not an apparatus that supplies the polymer to only the concave section 15b.

Furthermore, because of rotation of the suction drum 40, when the concave section 15b passes over the superabsorbent-polymer supplied region, only pulverized pulp P is supplied again from above and accumulates in the mesh pattern 48 (C region in FIG. 6). At this stage, pulverized pulp P is also supplied onto the superabsorbent polymer accumulated layer 13a in the concave section 15b, and a portion of three-layer structure are formed that has the two pulp accumulated layers 13b positioned at top and bottom and the superabsorbent polymer accumulated layer 13a. The portion having three-layer structure becomes the high-density region 13 in which the density of the superabsorbent polymer S is higher than in other portions.

Thereafter, with rotation of the suction drum 40, the mesh pattern 48 moves to a position of a pulp scraping mechanism 44. Of the pulverized pulp P accumulated in the mesh pattern 48, pulverized pulp P that has been excessively accumulated (for example, pulverized pulp layered with a certain thickness or more in the mesh pattern 48) is scraped by the pulp scraping mechanism 44. In the foregoing processes, the absorbent body material 12 is formed in the mesh pattern 48.

With further rotation of the suction drum 40, when the mesh pattern 48 accommodating the absorbent body material 12 reaches the bottom of the suction drum 40, the absorbent body material 12 is removed from the mesh pattern 48 and passed to a next process. At this stage, since the side faces of the projection section 48a of the mesh pattern 48 are tapered surfaces as shown in FIG. 7, it is possible to remove the absorbent body material 12 from the mesh pattern 48 with keeping the shape of the material. In the absorbent body material 12 removed from the mesh pattern 48, the hole sections 15a are positioned apart from each other and the high-density regions 13 are formed adjacent to the hole sections 15a, as shown in FIG. 6.

The absorbent body material 12 removed from the mesh pattern 48 is placed on a suction conveyor 60 used for transporting things while sucking air downward (S104). The suction conveyor 60 includes a suction apparatus that is not shown, and air is sucked by the suction apparatus into the suction conveyor 60 (that is, in a direction indicated by letter F2 in FIG. 6). The absorbent body material 12 is transported to a certain transporting direction (a direction indicated by the arrow in FIG. 6) in a state in which the absorbent body material 12 is sucked toward the suction conveyor 60 due to suction force of the suction apparatus. On a placement face of the suction conveyor 60, the continuous strip-like thin paper 11 is supplied therebefore by a thin-paper supplying section 50, and the absorbent body material 12 is to be placed onto the thin paper 11. That is, the absorbent body material 12 is attracted towards the suction conveyor 60 through the thin paper 11, and is transported together with the thin paper 11 in the transporting direction. Besides, since the absorbent body material 12 that has been formed in the mesh pattern 48 is consecutively placed on the suction conveyor 60, a plurality of the absorbent body materials 12 are placed on the suction conveyor 60 at intervals, as shown in FIG. 6. In the present embodiment, when the absorbent body material 12 is removed from the mesh pattern 48, the front face and the back face of the absorbent body material 12 reverse and the absorbent body material 12 is transported in a state in which, among surfaces of the absorbent body material 12, a surface to be the skin-contacting side in the completed absorbent article 1 opposes the placement face of the suction conveyor 60. However, the invention is not limited thereto. The absorbent body material 12 may be transported in a state in which a surface to be the skin-contacting side faces a side opposite the suction conveyor 60 side (that is, upward).

After the absorbent body material 12 is formed, the thin paper 11 existing between the absorbent body material 12 and the suction conveyor 60 is bent in such a manner as to wrap the absorbent body material 12, and covers the absorbent body material 12 (S106). In the present embodiment, a thin-paper bending section (not shown) is provided on the surface of the suction conveyor 60 in order to bend the thin paper 11. The thin paper 11 is bent when passing the thin-paper bending section and covers the absorbent body material 12. At this stage, since end sections of the thin paper 11 in the width direction (in a direction intersecting the transporting direction) overlap on an upper face (the non-skin-contacting face) side of the absorbent body material 12 and are affixed to each other. Thus, the thin paper 11 becomes tubular.

Next, in order to integrate the thin paper 11 and the absorbent body material 12, a certain portion of the absorbent body material 12 covered with the thin paper 11 is compressed, and an absorbent-body embossing process in which the absorbent body embossing is formed is performed (S108). The absorbent-body embossing process is performed by passing items between two rollers opposing vertically (not shown). For example, on a lower roller, a protrusion having a certain shape is formed at a portion contacting a region where an absorbent body embossing is formed when the absorbent body material 12 and the thin paper 11 are transported. A surface of an opposing upper roller is formed flat. The absorbent body material 12 wrapped with the thin paper 11 pass between the two rollers, and thereby the protrusion compresses both of the thin paper 11 and the absorbent body material 12 together. The thin paper 11 and the absorbent body material 12 are compressed by the protrusion and integrated by forming a plurality of the absorbent body embossings; the absorbent body 10 is made. In the present embodiment, it is preferable that the absorbent-body embossing process is performed onto areas other than a section corresponding to the hole section 15a in the absorbent body 10. This is because, when the vicinity of the hole section 15a is compressed by the protrusion, the thin paper 11 will break since the rigidity in the vicinity of the hole section 15a is lower than in a portion apart from the hole section 15a, as mentioned above.

Thereafter, the thin paper 11 and the absorbent body material 12 that are integrated (that is, the absorbent body 10) are severed with an absorbent-body cutter (not shown) along the external shape of the absorbent body material 12 (S110). The absorbent-body-production step S100 ends when the above-mentioned steps (S102 to S110) has been completed.

### Regarding Effectiveness of Absorbent Article according to Present Embodiment

With the absorbent article 1 of the present embodiment, the high-density regions 13 in which the density of the superabsorbent polymer S is higher than in a portion of the base material 15 are each provided at a position adjacent to each of the hole sections 15a. Therefore, even if the superabsorbent polymer S absorbs fluid and swells so that a volume of the superabsorbent polymer S increases, it is possible to accommodate the increased volume in the hole section 15a. Accordingly, it is possible to prevent a protuberance from occurring on an outer surface of the absorbent article 1 when the superabsorbent polymer S absorbs fluid and swells.

Further, if the absorbent article 1 absorbs fluid, a swelling superabsorbent polymer S exists in the hole section 15a. The swelling superabsorbent polymer S becomes difficult to absorb fluid, and it becomes impossible to permeate fluid in region where swelling superabsorbent polymers S are connected. For example, if superabsorbent polymers S exist throughout the absorbent body material 12, especially, superabsorbent polymers S exist in layers on the skin-contacting side of the absorbent article 1, the superabsorbent polymers S hold fluid and swell and superabsorbent polymers S are connected so that the fluid is inhibited from moving toward the non-skin-contacting side. As a result, the absorbed fluid remains in the skin-contacting side of the absorbent body material 12, and it becomes difficult to absorb fluid on the skin-contacting side; there is a risk that absorbency of the absorbent article 1 deteriorates. For this reason, a plurality of the hole sections 15a are positioned apart from each other and the high-density regions 13 are each provided adjacent to each of the hole sections 15a. Thereby, even when superabsorbent polymer S in the high-density region 13 swells, pulverized pulp P exists between the swelling superabsorbent polymers S. Accordingly, by causing fluid to widely penetrate with pulverized pulp P and causing superabsorbent polymer S positioned apart from each other to absorb fluid, it is possible to provide the absorbent article 1 having high absorbency. Further, the hole sections 15a that can excellently absorb fluid repeatedly are positioned apart from each other, and superabsorbent polymer S exists on an inner wall of the hole section 15a. Therefore, an effect can be achieved that superabsorbent polymer S absorbs moisture between skin and the absorbent body 10 when being worn and makes it difficult to confine moisture.

Further, since each high-density region 13 is adjacent to the hole section 15a in a specific direction, a plurality of the formed high-density regions 13 inhibit the swelling superabsorbent polymers S from being connected. Therefore, it is possible to suppress absorbency deterioration.

Further, the absorbent body material 12 made of an absorbent material, and the thin paper 11 for covering the absorbent body material 12 are provided. Therefore, it is possible to prevent superabsorbent polymer S of the high-density region 13 from flowing outside, and to form the high-density region 13 that is adjacent to the hole section 15a and in which the density of superabsorbent polymer S is higher than in other portions.

Further, with the method for producing the absorbent article 1 of the present embodiment, as well as the absorbent body material (hydrophilic fiber region) 12 and the hole section 15a are formed, superabsorbent polymer S is supplied to the concave section 15b that is formed adjacent to the hole section 15a in the absorbent body material 12. Therefore, it is possible to surely arrange superabsorbent polymer S adjacent to the hole section 15a.

In this case, pulverized pulp P supplied from the front end side of the formed absorbent body material 12 is inhibited from being supplied to the rear end side with respect to the projection section 48a, because of the projection section 48a projected from the bottom section 48c of the mesh pattern 48. Thus, supplying pulverized pulp P to the mesh pattern 48 from the front end side of the absorbent body material 12 is all that is required to allow the concave section 15b to be formed easily on the rear end side of the absorbent body material 12 with respect to the projection section 48a. When removed from the mesh pattern 48, the hole section 15a is formed at a position of the projection section 48a. Therefore, supplying pulverized pulp P from the front end side of the formed absorbent body material 12 with respect to the mesh pattern 48 is all that is required to form the concave section 15b adjacent to the hole section 15a.

Further, since pulverized pulp P and superabsorbent polymer S are supplied from above the suction drum 40 on whose outer circumferential face the mesh pattern 48 is provided, it is possible to supply the pulverized pulp P and the superabsorbent polymer S to the mesh pattern 48 while changing the orientation of the mesh pattern 48 with rotation of the suction drum 40. In other words, the pulverized pulp P and the superabsorbent polymer S can be supplied while changing a direction of the supply with respect to the mesh pattern 48 with rotation of the suction drum 40.

Further, a mold for forming the absorbent body material 12 is the mesh pattern 48 formed of net, and a drum on which the mesh pattern 48 is provided is the suction drum 40 that sucks air inward. Therefore, the pulverized pulp P and superabsorbent polymer S supplied from the certain direction can efficiently be collected into the mesh pattern 48 with air that is sucked toward the inside of the suction drum 40.

With rotation of the suction drum 40, the mesh pattern 48 moves, after the concave-section-forming step, to a position where superabsorbent polymer S can be supplied to the concave section 15b from above. Therefore, rotation of the suction drum 40 allows superabsorbent polymer S to surely be supplied to the concave section 15b formed in the concave-section-forming step. Particularly, since in the concave section 15b, which is formed adjacent to the projection section 48a, an amount of accumulated pulverized pulp P is less than in other portions (a weight is lower), suction force in the concave section 15b is stronger than in other portions. Thus, in the superabsorbent-polymer supplied region, where superabsorbent polymer S can be supplied with rotation of the suction drum 40, superabsorbent polymer S is more likely to be sucked than in other portions. Accordingly, it is possible to efficiently supply superabsorbent polymer S to the concave section 15b.

### Other Embodiments

Above, based on the above embodiments, the absorbent article and the method for producing the absorbent article according to the invention are mainly described. However, the above embodiments of the invention are for facilitating understanding of the invention, and are not limiting of the invention. The invention can of course be altered and improved without departing from the gist thereof, and equivalents are intended to be embraced therein. In particular, embodiments of the invention are not limited by the values or the material qualities of each material described in the above description. For example, in the above-mentioned embodiments, the pulverized pulp is described as an example of a "hydrophilic fiber". However, as other hydrophilic fibers, it is acceptable to use cellulose such as cotton, regenerated cellulose such as rayon or fibril rayon, semisynthetic cellulose such as acetate or triacetate, a fibrous polymer, thermoplastic hydrophobic chemical fiber, and the like. It is also acceptable that in the absorbent body material 12, granular deodorant, granular antibacterial material, granular coolant and the like gathers densely in addition to the pulverized pulp and the superabsorbent polymer S. Further, in the above-mentioned embodiments, the thin paper 11 such as a tissue paper is described as an example of a "covering member". However, as other covering members, it is acceptable to use woven fabric or nonwoven fabric made of cellulose such as cotton, regenerated cellulose such as rayon or fibril rayon, semisynthetic cellulose such as acetate or triacetate, fibrous polymer, thermoplastic hydrophobic chemical fiber, and the like. Besides, the absorbent body material 12 does not necessarily have to be covered.

In the above-mentioned embodiments, the fluid-permeable surface sheet 20 covering the surface of the absorbent body 10 on the skin-contacting side is described merely as a sheet member. However, a sheet member having irregularities on a surface of a surface sheet may be used, for example. If the sheet member having irregularities on the surface is used as a surface sheet, even when protuberances caused by swelling of the superabsorbent polymer S cannot be prevented sufficiently, it is possible to better prevent a wearer from having foreign-body sensation by absorbing with a concave section of the surface sheet a portion where protuberances can not be prevented. An example of a sheet member having irregularities on the above-mentioned surface includes, for example, a nonwoven fabric that has undergone airflow processing and has irregularities on a surface thereof by the following method: while transporting fibrous web in which fibers having been spun are deposited, by ejecting airflow onto a surface of the fibrous web from a plurality of nozzles, blowing apart fibers located at positions exposed to the airflow to move the fiber, and thereby forming valley sections at positions exposed to the airflow and mountain sections at positions not exposed to the airflow; the nozzles being arranged at intervals in a direction intersecting the transporting direction. That is, on the surface of the nonwoven fabric, the mountain sections and the valley sections are provided in such a manner as to extend along the longitudinal direction and be located side by side in the width direction. A basis weight of fibers in a slope section that connects a peak section of the mountain section and a bottom section of the valley section is larger than a basis weight of fibers in the peak section. In addition, the basis weight of fibers in the peak section is larger than a basis weight of fibers in the bottom section. Since the mountain section and the valley section is formed in this manner, if receiving fluid on the surface of the surface sheet, the fluid quickly passes the slope section having the large basis weight, and quickly moves to the absorbent body from the bottom section of valley section having the small basis weight. The better forwarding properties for forwarding fluid from the surface sheet to the absorbent body can be achieved. In addition, since the slope section has the large basis weight, the surface sheet is not crushed easily and inter-fiber empty spaces therein are maintained even when the body pressure of a wearer is exerted on the absorbent article. As a result, a state of excellent fluid forwarding properties can be maintained in the surface sheet.

Further, in the above-mentioned embodiments, for the sake of description, the configuration is described in which the absorbent body 10 has one absorbent body material 12 in the central region in the width direction. However, the invention is not limited thereto. For example, a configuration is acceptable in which both end sections of the absorbent body 10 in the width direction each have a side absorbent body in the longitudinal direction. Further, a configuration is acceptable in which the both end sections each have a solid gather instead of the side absorbent body. Besides, in the above-mentioned embodiments, the absorbent article 1 is folded in three. In other words, the case in which the absorbent article 1 has two folding line positions therein is described. However, the invention is not limited thereto. For example, the absorbent article 1 may be able to be folded in four.

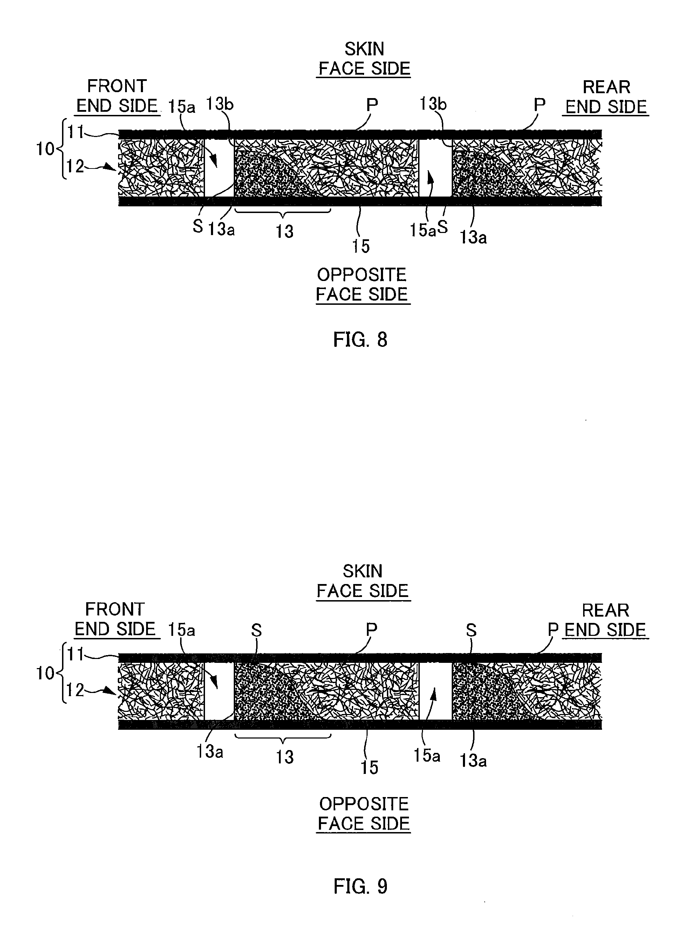

In the above-mentioned embodiments, the high-density region 13 has a three-layer structure in which a superabsorbent polymer layer is provided between two pulp accumulated layers 13b. However, the invention is not limited thereto. FIG. 8 is a cross-sectional view for illustrating a high-density region having the two-layer structure. FIG. 9 is a cross-sectional view for illustrating a high-density region having the one-layer structure.

For example, two-layer structure is also acceptable in which the pulp accumulated layer 13b and the superabsorbent polymer accumulated layer 13a superpose in the thickness direction of the absorbent body material 12 as shown in FIG. 8. In this case, this structure can be realized by arranging the superabsorbent-polymer supplied region where the superabsorbent polymer S is supplied from the superabsorbent-polymer supplier 52 further upstream in the rotational direction of the suction drum 40 than the position in the above-mentioned embodiments, and by increasing a speed of supplying the superabsorbent polymer S. Further, the configuration is acceptable in which the high-density region 13 has only the superabsorbent polymer accumulated layer 13a without the pulp accumulated layer 13b as shown in FIG. 9. In this case, this structure can be realized by arranging the superabsorbent-polymer supplied region where the superabsorbent polymer S is supplied from the superabsorbent-polymer supplier 52 further upstream in the rotational direction of the suction drum 40 than in the case of the two-layer structure, and by further increasing the speed of supplying the superabsorbent polymer S.

Further, in the above-mentioned embodiments, a configuration is described in which the superabsorbent polymer S is supplied from above the suction drum 40 in the same manner as the pulverized pulp P. However, the invention is not limited thereto. For example, a base material including a hole section and a concave section adjacent to the hole section may be formed on the suction drum, and on the suction conveyor, superabsorbent polymer S may be supplied to the concave section. In this case, rotation of a patterned roller on whose outer circumferential face recesses for holding superabsorbent polymer S are positioned apart from each other is synchronized with the base material transporting by the suction conveyor. And, at a position where the recesses of the patterned roller and the concave section of the base material oppose, superabsorbent polymer S is sucked toward the base material and is supplied into the concave section. Here, the base material refers to an absorbent body material that does not have superabsorbent polymer S in its concave section.

Further, in the above-mentioned embodiments, an example is described in which the absorbent body material 12 is formed using the suction drum 40 on whose outer circumferential face the mesh pattern 48 is recessed. However, the suction drum 40 does not have to be used. For example, this method is also acceptable in which air is sucked to below a mesh pattern that is positioned flat, pulverized pulp is supplied from above and a side of the mesh pattern, a base material having a concave section adjacent to a hole section is formed, and thereafter an absorbent body material is formed by supplying superabsorbent polymer S to a concave section with the patterned roller or the like.

Further, in the above-mentioned embodiments, the hole sections 15a and the high-density regions 13 are positioned apart in both end sections of the absorbent body material 12 in the longitudinal direction, and does not exist in the central section. However, the invention is not limited thereto. The hole sections and the high-density regions may exist in the central section in the longitudinal direction.

Further, in the above-mentioned embodiments, the hole section 15a is formed in an oval shape on the plane defined by in the longitudinal direction and the width direction of the absorbent body material 12. However, a circle, a quadrilateral, a triangle, and the like are also acceptable, and the invention is not limited to the oval shape.

Further, in the above-mentioned embodiments, an example in which pulverized pulp is accumulated into the mesh pattern 48 is described as step S102 in which the absorbent body material 12 is formed. However, a method for forming the absorbent body material 12 is not limited thereto. For example, as another method, a method can be considered in which a sheet having layers of pulp fiber and thermoplastic fiber (both are hydrophilic fiber, and hereinafter collectively referred to merely as fiber) is used and a process for providing the hole section 15a and the concave section 15b adjacent to the hole section 15a is performed on the sheet.

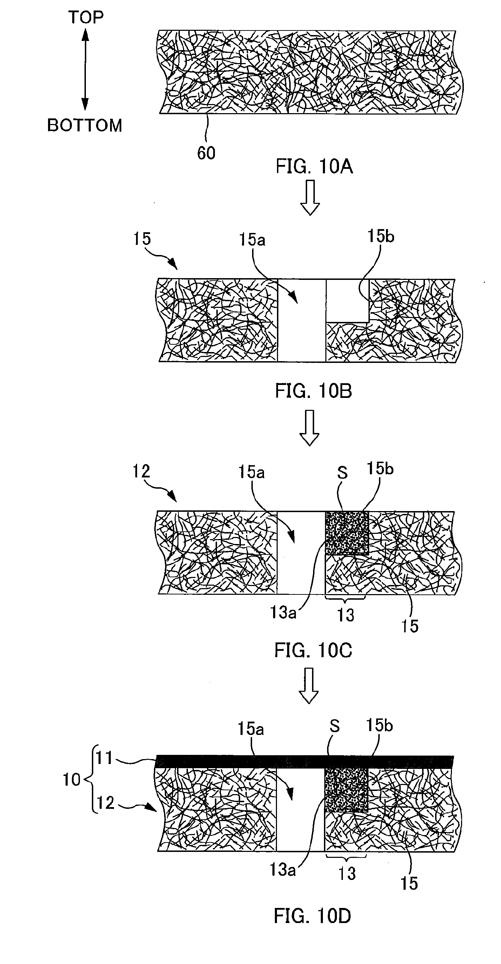

A producing method using a sheet of hydrophilic fiber is described below with reference to FIGS. 10A to 10D. FIGS. 10A to 10D are diagrams for illustrating a process in which the base material 15 of the absorbent body material 12 is made of a sheet (hereinafter referred to as an air-laid sheet), and the absorbent body 10 is formed using the base material 15 that has been made. FIG. 10A is a cross-sectional view of an unprocessed air-laid sheet. FIG. 10B is a cross-sectional view showing a base material having a hole section and a concave section formed therein. FIG. 10C is a cross-sectional view showing an absorbent body material to whose concave section superabsorbent polymer S has been supplied. FIG. 10D is a cross-sectional view showing an absorbent body in which thin paper and the absorbent body material are integrated. FIGS. 10A to 10D each show an enlarged schematic diagram of a vertical cross section of a portion corresponding to the hole section 15a and the high-density region 13.

In the case of using an air-laid sheet 54, first, the substantially flat air-laid sheet 54 shown in FIG. 10A is severed so as to correspond to an external shape of the base material 15 and the hole section 15a serving as a through hole is formed. Thereafter, a compression-bonding embossing process is performed on the air-laid sheet 54 having the hole section 15a formed thereon, so as to form the concave section 15b adjacent to the hole section 15a. The compression-bonding embossing process is a process in which, in a similar manner to the above-mentioned groove-embossing and absorbent-body embossing process, by passing items between two rollers opposing vertically, protrusions provided on one of the rollers compresses the air-laid sheet 54 to form embossing. As shown in FIG. 10B, the concave section 15b is formed by the compression-bonding embossing process, and the base material 15 is made of the air-laid sheet 54. In an example of the present producing method, the air-laid sheet 54 is severed and the hole section 15a is formed before performing the compression-bonding embossing process. However, after performing the compression-bonding embossing process, the air-laid sheet 54 may be severed and the hole section 15a may be formed. Further, the base material 15 may be formed in one process by pressing the air-laid sheet 54 with a member that includes a cutter for severing the air-laid sheet 54 along the external shape of the absorbent body material 12, a cutter for forming the hole section 15a, and a projection section for forming the concave section 15b.

As shown in FIG. 10C, while the base material 15 formed of the air-laid sheet 54 is transported in the transporting direction with placing on the transporting apparatus, superabsorbent polymer S is supplied to the concave section 15b of the base material 15.

Thereafter, the absorbent-body embossing process is performed in which the upper face of the absorbent body material 12 is covered with the thin paper 11 to integrate the thin paper 11 and the absorbent body material 12. Then, the absorbent body 10 shown in FIG. 10D is finished. In an example in which a sheet is used, the air-laid sheet 54 in which pulp fiber and thermoplastic fiber are layered is used. However, an air-laid sheet in which superabsorbent polymer S and fiber mix may be used. In this case, it is necessary to adjust a mixing rate of superabsorbent polymer S in the air-laid sheet to 40% or less (more preferably 20% or less).

## Claims

1. An absorbent article, comprising:
a fluid-permeable surface sheet;
a fluid-impermeable back face sheet; and
a liquid-absorbent core that is between the surface sheet and the back face sheet and that includes hydrophilic fiber and superabsorbent resin,
the liquid-absorbent core having a plurality of through holes formed thereon and high-density regions, the high-density regions being each provided at a position adjacent to each of the through holes, a density of the superabsorbent resin in each of the high-density regions being higher than in another region.

2. An absorbent article according to claim 1, wherein
the liquid-absorbent core is substantially rectangular, and
a plurality of the through holes are provided in both a longitudinal direction and a width direction of the liquid-absorbent core.

3. An absorbent article according to claim 1 or 2, wherein
the high-density region is adjacent to the through hole on at least either one of both sides of the through hole in the longitudinal direction of the liquid-absorbent core.

4. A method for producing an absorbent article, comprising:
a concave-section-forming step in which hydrophilic fiber is supplied to a mold having a projection section that projects from a bottom section and that is used to form a through hole extending in a front-back face direction, from a certain direction with respect to the mold, and in which a hollowed concave section is formed on an opposite side to a side of the projection section on which the hydrophilic fiber is supplied; and
a supplying step in which superabsorbent resin is supplied to the concave section.

5. A method for producing an absorbent article, according to claim 4, wherein
the mold is recessed on an outer circumferential face of a drum that rotates, and
the hydrophilic fiber is supplied from above the drum to the mold whose orientation changes with rotation of the drum.

6. A method for producing an absorbent article, according to claim 5, wherein
after the concave-section-forming step, the mold is moved by rotation of the drum to a position at which the superabsorbent resin can be supplied to the concave section from above.
